(19) Europäisches Patentamt

European Patent Office

Office européen des brevets

(11) Publication number: **0 295 962 B1**

# EUROPEAN PATENT SPECIFICATION

(45) Date of publication of patent specification: **24.11.93** (51) Int. Cl.[5]: **A61K 35/84**

(21) Application number: **88305583.2**

(22) Date of filing: **17.06.88**

(54) Protein-polysaccharide for treating retroviral infections.

(30) Priority: **18.06.87 JP 152084/87**

(43) Date of publication of application:
**21.12.88 Bulletin 88/51**

(45) Publication of the grant of the patent:
**24.11.93 Bulletin 93/47**

(84) Designated Contracting States:
**BE DE FR GB IT NL**

(56) References cited:
**EP-A- 0 022 426**
**FR-A- 2 335 235**

**COMP. IMMUN. MICROBIOL. INFECT. DIS.,**
**vol. 9, no. 2/3, 1986, Pergamon Journals Ltd**
**(GB); J.-F.BACH, pp. 233-236&NUM;**

**ARCH DERMATOL, vol 123, June 1987;**
**M.DUVIC et al., pp. 751-756&NUM;**

**J.Drews, IMMUNPHARMAKOLOGIE, Springer-**
**Verlag; pp. 174-175&NUM;**

**MICROBIOL. IMMUNOL., vol. 31 (I), 1987;**
**M.OKADA et al., pp. 45-57&NUM;**

(73) Proprietor: **KUREHA KAGAKU KOGYO**
**KABUSHIKI KAISHA**
**1-9-11, Nihonbashi, Horidome-cho**
**Chuo-ku Tokyo 103(JP)**

(72) Inventor: **Hirose, Kunitaka**
**3-26-1 Hyakunin-cho**
**Shinjuku-ku**
**Tokyo(JP)**
Inventor: **Furusho, Takao**
**1-6-13 Asahi-machi**
**Michida-shi Tokyo(JP)**
Inventor: **Muto, Shigeaki**
**3-23-2, Higashi-Horikiri**
**Katsushika-ku Tokyo(JP)**
Inventor: **Niimura, Koichi**
**6-104 Shin-Sayama-Haitsu**
**63 Aoyagi**
**Sayama-shi Saitama-ken(JP)**
Inventor: **Oohara, Minoru**
**19-25 Fujima-cho**
**Itabashi-ku**
**Tokyo(JP)**
Inventor: **Oguchi, Yoshiharu**
**306 Koopo-Cherii**
**3-10-23 Kasuga-cho**
**Nerima-ku Tokyo(JP)**

**BIOCHEMICAL AND BIOPHYSICAL RE-
SEARCH COMMUNICATIONS, vol. 148, no. 2,
October 1987, Academic Press Inc. (US);
T.S.TOCHIKURA et al., pp. 726-733&NUM;**

**BIOCHEM. BIOPHYS. RESEARCH COMMUNI-
CATIONS, vol. 149, no. 2, December 1987,
Academic Press Inc. (US); K.HIROSE et al.,
pp. 562-567&NUM;**

Inventor: **Matsunaga, Kenichi**
**989-17 Kami-Arai**
**Tokorozawa-shi Saitama-ken(JP)**
Inventor: **Kakuchi, Junji**
**6-16-30-406 Minami-Karasuyama**
**Setagaya-ku Tokyo(JP)**
Inventor: **Sugita, Norifumi**
**2-10-13-402 Kasuga**
**Bunkyo-ku**
**Tokyo(JP)**
Inventor: **Yoshikumi, Chikao**
**2-19-46 Higashi**
**Kunitachi-shi Tokyo(JP)**
Inventor: **Takahashi, Masaaki**
**1-5-33-314 Takanawa**
**Minato-ku Tokyo(JP)**

(74) Representative: **Woods, Geoffrey Corlett et al**
**J.A. KEMP & CO.**
**14 South Square**
**Gray's Inn**
**London WC1R 5EU (GB)**

## Description

The present invention relates to the use of a protein-polysaccharide derived from a basidiomycete belonging to the genus Coriolus.

New types of viral diseases such as B-type hepatitis, adult T-cell leukemia and AIDS have attracted attention in recent years. Preventive vaccination has been used to combat viral diseases. This has succeeded in eradicating, or considerably reducing instances of, such diseases as smallpox, yellow fever and polio. However, vaccination is insufficient to cure diseases involving persistent or latent infection such as AIDS. The development of an anti-retroviral drug, which is safe and shows an excellent effect against such diseases, has been desired.

Some of the present inventors have previously proposed many inventions relating to protein-polysaccharides derived from the basidiomycetes. Such inventions are disclosed in US-A-4,051,314, 4,202,885, 4,289,688, 4,271,151, 4,202,969, 4,229,570, 4,140,578, 4,237,233, 4,288,555, 4,162,939, 4,159,225, 4,268,505 and 4,663,438; and GB-A-1,331,513 and 1,581,315.

FR-A-2335235 discloses the preparation of an antitumour nitrogen-containing polysaccharide by extracting a basidiomycete fungus which may be of the genus Coriolus. EP-A-0022426 is concerned with a nasal preparation for the prevention of infections caused by viruses of the respiratory tract. The active ingredient of the preparation is one or more nitrogen-containing polysaccharides obtained from fungi.

Bach, Comp. Immun. Microbiol. Infect. Dis. 9, 2/3, 233-236, 1986 is concerned with possible clinical indications of immunostimulants. A long list of immunostimulants includes KRESTIN (Trade Mark). Okada and Minamishima, Microbiol. Immunol. 31(1), 45-47, 1987 investigated the host-mediated antiviral effect of two biological response modifiers, OK-432 and PS-K (KRESTIN) against murine cytomegalovirus in normal and immunologically deficient mice of the same litters.

It has now been found that the adsorption of human immunodeficiency virus (HIV), a retrovirus, by human lymphocytes is inhibited when the HIV is treated with a protein-polysaccharide derived from a basidiomycete belonging to the genus Coriolus, and that the protein-polysaccharide can appreciably inhibit the activity of reverse transcriptase which is essential for the replication of the virus.

Accordingly, the present invention provides the use of a protein-polysaccharide in the preparation of a medicament for use as an antiretroviral agent, which protein-polysaccharide is obtainable by extracting with hot-water or an aqueous alkaline solution the mycelium or fruit body obtained from a culture of a basidiomycete belonging to the genus Coriolus and has the following properties:

(i) a molecular weight of from 5,000 to 300,000 (as measured by ultracentrifugal analysis);

(ii) a protein content of 18 to 38% by weight, the protein component having a nitrogen content of 3 to 6%;

(iii) the polysaccharide portion is composed of $\beta$-D-glucan units which have a branched structure containing $1 \to 3$, $1 \to 4$, and $1 \to 6$ bonds;

(iv) not less than 36% of the protein-component amino acids are the acidic amino acids aspartic acid and glutamic acid and the and neutral amino acids valine and leucine while not more than 8% of the protein-component amino acids are the basic amino acids lysine and arginine;

(v) the protein-polysaccharide is soluble in water but hardly soluble in methanol, pyridine, chloroform, benzene and hexane; and

(vi) the protein-polysaccharide begins to decompose when heated to 120°C.

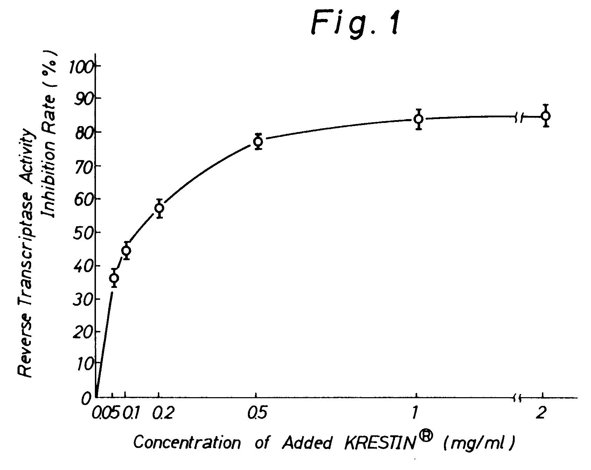

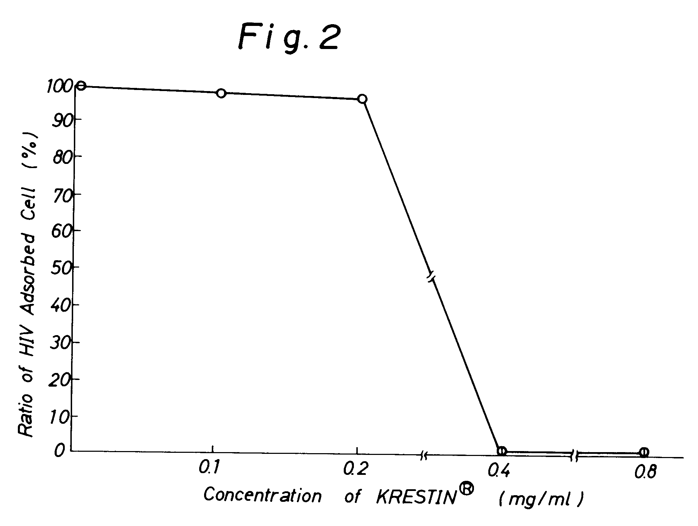

The protein-polysaccharide can therefore be used to treat retroviral infections such as AIDS. In the drawings FIG. 1 shows the reverse transcriptase activity inhibition rate by KRESTIN, and FIG. 2 shows the inhibitory activity of KRESTIN against adsorption of HIV on lymphocytes.

The protein-polysaccharide for use according to the present invention is derived from the mycelia of a fungus belonging to the genus Coriolus (FERM-P2412 (ATCC 20547)) and commercially sold under the trade mark of KRESTIN (see "Saikin no Shinyaku" (Latest New Drugs), Vol. 28, pp. 14-16 (1977) and Vol. 29, pp. 96-101 (1978); "Iyakuhin Yoran" (Handbook of Pharmaceuticals), p. 1,346, May 1979, 6th ed., Yakugyo Jihosha; and "Iryoyaku, Nippon Iyakuhin Shu" (Drugs, A Collection of Japanese Pharmaceuticals), 7th ed., p. 240 (1983) Yakuji Jihosha). The following description gives a glimpse of the characteristic properties of KRESTIN.

The polysaccharide portion of the main fraction is composed of $\beta$-D-glucan, and this glucan portion has a branched structure containing $1 \to 3$, $1 \to 4$ and $1 \to 6$ bonds. This polysaccharide contains protein with a nitrogen content of 3 - 6%. The protein-component amino acids are mostly acidic amino acids such as aspartic acid or glutamic acid, and neutral amino acids such as valine or leucine. Basic amino acids such as lysine or arginine, are small in quantity. This protein-polysaccharide is soluble in water but scarcely soluble in methanol, pyridine, chloroform, benzene and hexane, and begins to decompose when heated to about

3

EP 0 295 962 B1

120°C.

KRESTIN contains about 18 to 38% by weight of protein and has a molecular weight of 5,000 to 300,000 (as determined by ultracentrifugal analysis).

As stated above, KRESTIN has already been offered to society as an anticancer drug, and it is very low in toxicity, has no possibility of causing disturbance of intestinal flora and is capable of long-term administration. Also, it gives no influence to mutagenecity or allergic reactions, so that it has no risk of causing a teratogenetic activity or allergic reactions and is thus a very safe substance for medical use.

The protein-polysaccharide used according to the present invention (hereinafter referred to as the present substance) inhibits the adsorption of human immuno-deficiency virus (HIV), which is a kind of retrovirus, on human lymphocytes and also noticeably inhibits the activity of reverse transcriptase which is essential for the replication of the virus. These attest to the high potency of the present substance for the treatment of retroviral infectious diseases such as acquired immunological deficiency syndrome (AIDS) caused by HIV.

The present substance is very low in its toxicity and produces almost no harmful side effects, and thus it is known as a substance which is very safe to the living organisms. The acute toxicity of the present substance is shown in Table 1 below.

Table 1

| Animal tested | Route of administration | $LD_{50}$ (mg/kg) | |
|---|---|---|---|
| | | Female | Male |
| Mouse | Oral | > 20000 | > 20000 |
| Rat | Oral | > 20000 | > 20000 |

The acute toxicity shown in the above table was determined by the following test method.

Used as test animals were ICR-JCL strain mice which were 4 to 5 weeks old and weighed 21 to 24 g, and Donryu strain rats which were 4 to 5 weeks old and weighed 100 to 150 g. The present substance was dissolved in a physiological saline solution and administered to the test animals orally. The general symptoms, death and body weight of each test animal were observed over a period of 7 days, and after the end of this observation period, each test animal was killed and anatomized.

As seen from Table 1, there was caused no death of rats and mice even when they were given the largest possible dosage of the present substance, and it was practically impossible to determine the $LD_{50}$ value. This indicates such high safety of the present substance for the animal organism.

As seen from the above, the present substance is very low in acute toxicity and useful as an antiretroviral drug showing an inhibiting activity against retroviral infection and safe in use. Thus, the present substance is effective for the treatment of retroviral infectious diseases.

In use of the present substance as an antiretroviral drug, the present substance can be worked into any desired form of preparation (pharmaceutical composition). Also, the preparation can be administered in any desired way, either oral or parenteral. Further, the pharmaceutical composition according to the present invention is not reduced in its efficacy even when used in combination with other known antiretroviral drugs such as azido-3'-dioxythymidine (AZT). Thus, use of the present substance in combination with other known drugs such as mentioned above proves to be an effective means for the treatment of retroviral infectious diseases.

In the case of oral administration, the preparation may take the form of tablet, granules, powder or capsule, which may contain in its composition carrier(s) or adjuvant(s) generally used for the pharmaceutical composition, such as binder, inclusion, excipient, lubricant, disintegrator or wetting agent. When used as an oral liquid preparation, the composition of the present invention may be worked into a liquid for internal use, shake mixture, suspension, emulsion or syrup. Also, the composition of the present invention may take the form of a dry product which is made into a solution when used. Such liquid preparations may contain generally used types of additives and/or preservatives.

In the case of injection, the composition of the present invention may contain such additives as stabilizer, buffering agent, preservative or isotonizing agent, and the preparation may be offered in the form of unit dosage ampule or in the multiple-dosage containers. The composition of the present invention may be worked into the form of aqueous solution, suspension, solution or emulsion in an oleaginous or aqueous vehicle. The present substance as the active ingredient may be in the form of powder which is redissolved in a suitable vehicle such as pyrogen-free sterilized water before use.

4

The antiretroviral drug of the present invention is administered to the man and animals either orally or parenterally. Sublingual administration is included in the form of oral administration. The form of the parenteral administration include injection such as subcutaneous, intramuscular and intravenous injection, and instillation.

The dosage of the antiretroviral drug of the present invention is variable depending on whether the subject is man or animal and according to such factors as age, individual difference or condition of the disease, but usually when the subject is man, the oral dosage of the present substance is 10 to 1,000 mg, preferably 20 to 600 mg per 1 kg of body weight and per day, and this amount of the present substance is given in one to three portions.

The present substance is a pharmaceutical substance which is extremely high in safety and efficacious against the retroviral infectious diseases caused by HIV, such as AIDS (acquired immunological deficiency syndrome).

The present invention will hereinafter be described more particularly with reference to the examples thereof, however the examples are merely intended to be illustrative and not to be construed as limiting the scope of the invention.

EXAMPLE 1

Retrovirus has RNA as a gene and performs DNA synthesis with gene RNA as template by using its own reverse transcriptase in the infected cell. Reverse transcriptase is an enzyme peculiar to the retroviruses. In this Example, it was examined whether or not the present substance inhibits the activity of reverse transcriptase.

20 mg of KRESTIN was dissolved in 10 ml of sterilized distilled water and the solution was adjusted to a concentration of 2.0, 1.0, 0.5, 0.2, 0.1 or 0.05 mg/ml. 1 $\mu$l of 20 mM D.T.T. (dithiothreitol, produced by Sigma Co., Ltd.), 5 $\mu$l of a 5-fold concentrated enzyme reaction solution (250 mM Tris-HCl (pH 8.3), 250 mM KCl and 40 mM $MgCl_2$), 1 $\mu$l of a 3d NPT solution (1 mM dATP, 1 mM dGTP and 1 mM dTTP, produced by Sigma Co., Ltd.), 2 $\mu$l of a 100 $\mu$g/ml oligomer $(dT)_{12-18}$ (PL Biochemicals Co., Ltd), 1 $\mu$l of messenger RNA (derived from normal rat liver, 1 $\mu$g/$\mu$l), 0.5 $\mu$l of RNase inhibitor (16 unit/$\mu$l, Takara Shuzo KK) and 1 $\mu$l of $[\alpha\text{-}^{32}P]$ dCTP (up to 800 Ci/mmol, 10 $\mu$Ci/$\mu$l, Amasham Japan Co., Ltd.) were supplied into a 1.5 ml Eppendolf's tube and the Eppendolf's tube was placed in a water bath of 37°C.

5 minutes thereafter, 12.5 $\mu$l of the previously prepared 1 mg/ml KRESTIN solution was added into the reaction tube, followed by further addition of 1 $\mu$l of reverse transcriptase (derived from Rous associated virus, 7 unit/$\mu$l, produced by Takara Shuzo Kabushiki Kaisha) so that the final amount of the reaction solution would become 25 $\mu$l, and they were reacted at 37°C.

One hour thereafter, 5 $\mu$l of the reaction solution was infiltrated into 2 cm x 2 cm sheets of DEAE paper (made by Toyo Roshi Kabushiki Kaisha). After air drying, each sheet of filter paper was immersed in 10 ml of a 0.5 M $Na_2HPO_4$ solution and $[\alpha\text{-}^{32}p]$ dCTP which was not used for the DNA synthesis and remained on the filter paper was washed away under shaking. (This operation was conducted 5 times at a 5 minutes' interval).

Thereafter, the thus treated DEAE paper sheets were placed in a glass vial containing 10 ml of liquid scintillation cocktail (made by Amasham Japan Co., Ltd.) and the radioactivity (c.p.m.) of each DEAE paper sheet was counted for one minute by a scintillation counter (made by Aroka Co., Ltd.).

Similar specimens were prepared with the 2.0, 0.5, 0.2, 0.1 and 0.05 mg/ml KRESTIN solutions and similar measurements were made on these specimens.

The reverse transcriptase activity inhibition rate (%) was determined from the following formula:

$$\textbf{Reverse transcriptase activity inhibition rate (\%)} = \frac{Co - Cs}{Co} \times 100$$

Co:    radioactivity of the specimen when not added with the present substance
Cs:    radioactivity of the specimen when added with the present substance
The reverse transcriptase activity inhibition rate of KRESTIN is shown in FIG. 1.
As seen from FIG. 1, the present substance markedly inhibited the reverse transcriptase activity.

### EXAMPLE 2

Viruses are adsorbed on and penetrate into the target cell, and are replicated by making use of such target cell. In this Example, it was examined whether or not the present substance inhibits the adsorption of HIV on human lymphocytes.

1 ml of each suspension of human immunodeficiency virus (HIV-1) and 1 ml of each KRESTIN solution (0.1, 0.2, 0.4 or 0.8 mg/ml concentration) were put into a test tube and the test tube was placed still in ice. Two hours thereafter, 1 ml of virus suspension was taken out from each test tube and the virus was adsorbed on a human lymphocyte-derived cell strain MT-4 (Jpn. J. Cancer Res. (Gann), 28, 219-229 (1982)) at a multiplicity of infection (M.O.I.) approximately equal to 2. After centrifugal separation at 2,000 r.p.m. for 10 minutes, the supernatant was removed and the precipitated MT-4 cells were set free in RPMI 1640 (Gibco Laboratories, NY) containing 20% of FCS so that the cell concentration would become $2 \times 10^5$/ml. The MT-4 cell suspension was pipetted in portions of 100 ul into the 96-hole plates and cultured in the air under the conditions of 5% $CO_2$ and 37°C. On the 3rd day of culture, the HIV-1 adsorbed cells and non-adsorbed cells were calculated by using the indirect fluorescent antibody technique.

The MT-4 cells were fixed by a methanol treatment and reacted with the HIV-1-infected patient's antiserum at 37°C. 30 minutes thereafter, the cells were washed with PBS and reacted with fluorescein isocyanate-conjugated rabbit antihuman IgG (immunoglobulin) at 37°C.

500 MT-4 cells were observed under a fluorescence microscope and the fluorescent positive cells were calculated as HIV-1 adsorbed cells.

Ratio of HIV-1 adsorbed cell (%) =

$$\frac{\text{HIV-1 adsorbed cells}}{500 \text{ cells observed}} \times 100$$

The results are shown in FIG. 2. As seen from FIG. 2, adsorption of HIV-1 (HTLV-III) on lymphocytes was perfectly inhibited by the addition of 400 $\mu$g/ml or more of the present substance.

As described in Examples 1 and 2, the present substance has an inhibitory activity against adsorption of HIV-1 on human lymphocytes as well as a reverse transcriptase inhibiting activity, and is deemed to be efficacious against the HIV infected diseases such as AIDS.

### PREPARATION EXAMPLE

300 mg of KRESTIN was filled in the #0 hard capsules by using a pressure type automatic filling machine to obtain the capsules.

## Claims

1. Use of a protein-polysaccharide in the preparation of a medicament for use as an antiretroviral agent, which protein-polysaccharide is obtainable by extracting with hot-water or an aqueous alkaline solution the mycelium or fruit body obtained from a culture of a basidiomycete belonging to the genus Coriolus and has the following properties:

   (i) a molecular weight of from 5,000 to 300,000 (as measured by ultracentrifugal analysis);

   (ii) a protein content of 18 to 38% by weight, the protein component having a nitrogen content of 3 to 6%;

   (iii) the polysaccharide portion is composed of $\beta$-D-glucan units which have a branched structure containing 1 → 3, 1 → 4, and 1 → 6 bonds;

   (iv) not less than 36% of the protein-component amino acids are the acidic amino acids aspartic acid and glutamic acid and the and neutral amino acids valine and leucine while not more than 8% of the protein-component amino acids are the basic amino acids lysine and arginine;

   (v) the protein-polysaccharide is soluble in water but hardly soluble in methanol, pyridine, chloroform, benzene and hexane; and

   (vi) the protein-polysaccharide begins to decompose when heated to 120°C.

**2.** The use according to claim 1, wherein the medicament is for use against a human immunodeficiency virus (HIV).

**3.** The use according to claim 2, where the HIV is HIV-1.

**4.** The use according to claim 1, wherein the medicament is for use in treating AIDS.

**Patentansprüche**

**1.** Verwendung eines Protein-Polysaccharids bei der Herstellung eines Arzneimittels zur Verwendung als antiretrovirales Mittel, wobei dieses Protein-Polysaccharid durch Extrahieren des Mycels oder Fruchtkörpers, welche aus einer Kultur eines Basidiomyceten, der zu der Gattung Coriolus gehört, erhalten werden, mit heißem Wasser oder einer wäßrigen alkalischen Lösung erhalten werden kann und die folgenden Eigenschaften hat:

(i) ein Molekulargewicht von 5.000 bis 300.000 (gemessen durch Ultrazentrifugations-Analyse);
(ii) einen Proteingehalt von 18 bis 38 Gewichtsprozent, wobei die Proteinkomponente einen Stickstoffgehalt von 3 bis 6 Prozent hat;
(iii) der Polysaccharidteil ist aus $\beta$-D-Glucan-Einheiten zusammengesetzt, welche eine verzweigte Struktur haben, die $1 \rightarrow 3$, $1 \rightarrow 4$ und $1 \rightarrow 6$ Bindungen enthält;
(iv) nicht weniger als 36 Prozent der Aminosäuren der Proteinkomponente sind die sauren Aminosäuren Asparaginsäure und Glutaminsäure und die neutralen Aminosäuren Valin und Leucin, während nicht mehr als 8 Prozent der Aminosäuren der Proteinkomponente die basischen Aminosäuren Lysin und Arginin sind;
(v) das Protein-Polysaccharid ist in Wasser löslich, aber in Methanol, Pyridin, Chloroform, Benzol und Hexan kaum löslich; und
(vi) das Protein-Polysaccharid beginnt sich zu zersetzen, wenn es auf 120 ° C erwärmt wird.

**2.** Verwendung nach Anspruch 1, worin das Arzneimittel für die Verwendung gegen ein humanes Immundefizienz-Virus (HIV) ist.

**3.** Verwendung nach Anspruch 2, wobei das HIV HIV-1 ist.

**4.** Verwendung nach Anspruch 1, worin das Arzneimittel für die Verwendung bei der Behandlung von AIDS ist.

**Revendications**

**1.** Utilisation d'un polysaccharide protéinique dans la préparation d'un médicament à utiliser comme agent antirétroviral, lequel polysaccharide protéinique peut être obtenu en extrayant avec de l'eau chaude ou une solution alcaline aqueuse le mycélium ou le fruit obtenu à partir d'une culture d'un basidiomycète appartenant au genre *Coriolus*, et a les propriétés suivantes :

(i) un poids moléculaire de 5.000 à 300.000 (mesuré par analyse d'ultracentrifugation) ;
(ii) une teneur en protéine de 18 à 38 % en poids, le composant protéinique ayant une teneur en azote de 3 à 6 % ;
(iii) la partie polysaccharide est composée d'unités de $\beta$-D-glucane qui ont une structure ramifiée contenant des liaisons $1 \rightarrow 3$, $1 \rightarrow 4$, et $1 \rightarrow 6$ ;
(iv) pas moins de 36 % des acides aminés du composant protéinique sont les acides aminés acides, acide aspartique et acide glutamique, et les acides aminés neutres, valine et leucine, tandis que pas plus de 8 % des acides aminés du composant protéinique sont les acides aminés basiques, lysine et arginine ;
(v) le polysaccharide protéinique est soluble dans l'eau mais guère soluble dans le méthanol, la pyridine, le chloroforme, le benzène et l'hexane ; et
(vi) le polysaccharide protéinique commence à se décomposer lorsqu'on le chauffe à 120 ° C.

**2.** Utilisation selon la revendication 1, dans laquelle le médicament est à utiliser contre un virus de l'insuffisance immunitaire humaine (HIV).

**3.** Utilisation selon la revendication 2, où le HIV est le HIV-1.

**4.** Utilisation selon la revendication 1, dans laquelle le médicament sert à traiter le SIDA.

Fig. 1

Fig. 2